Europäisches Patentamt

European Patent Office

Office européen des brevets

(11) Publication number: **0 120 551**

**B1**

# EUROPEAN PATENT SPECIFICATION

(45) Date of publication of patent specification: **01.08.90**

(51) Int. Cl.⁵: **C 12 N 15/00,** C 12 N 1/16

(21) Application number: **84300091.0**

(22) Date of filing: **06.01.84**

(60) Divisional application 89106868.6 filed on 06/01/84.

(54) Yeast expression systems with vectors having a GAPDH promoter, and synthesis of foreign proteins.

(30) Priority: **22.02.83 US 468589**

(43) Date of publication of application:
**03.10.84 Bulletin 84/40**

(45) Publication of the grant of the patent:
**01.08.90 Bulletin 90/31**

(84) Designated Contracting States:
**AT BE CH DE FR GB IT LI LU NL SE**

(56) References cited:
EP-A-0 060 057
EP-A-0 072 318
EP-A-0 073 657
EP-A-0 077 689
EP-A-0 089 666
EP-A-0 096 910
EP-A-0 103 409
EP-A-0 109 559

(73) Proprietor: CHIRON CORPORATION
4560 Horton Street
Emeryville California 94608 (US)

(72) Inventor: Burke, Rae Lyn
1624 8th Avenue
San Francisco California (US)
Inventor: Tekamp/Olson, Patricia
1426 43rd Avenue
San Francisco California (US)
Inventor: Rosenberg, Steven
3861 Fruitvale Avenue
Oakland California (US)
Inventor: Valenzuela, Pablo D. T.
455 Upper Terrace No. 3
San Francisco California (US)

(74) Representative: Glawe, Delfs, Moll & Partner
Patentanwälte
Postfach 26 01 62 Liebherrstrasse 20
D-8000 München 26 (DE)

## Description

### Background of the invention

For maximal expression of foreign genes in microbial systems it is usually advantageous to empl⟨ homologous regulatory elements within the expression vector. Efficiency of expression (produ formation) is believed to be a function of and proportional to the strength of the promoter employed. addition, regulation of gene expression by nutritional factors under the control of the experimenter offers further useful manipulatory tool. The glycolytic enzyme genes of yeast, e.g., those coding f⟨ glyceraldehyde-3-phosphate dehydrogenase (GAPDH) and pyruvate kinase (PyK), possess the above usef properties, i.e., high levels of expression (and thus by inference very efficient promoters) and susceptibili to regulation by components of the growth medium. For example, GAPDH can comprise as much as 5% ⟨ the dry weight of commercial baker's yeast (Krebs, E. G., *J. Biol. Chem.* (1953) *200*:471). Furthermore, the⟨ enzymes are also highly inducible. For example, when yeast cultures are shifted from growth on acetate ⟨ glucose, the activity of GAPDH increased up to 200-fold in proportion to the concentration of the sugar i the medium (Maitra, P. K. and Lobo, Z., *J. Biol. Chem.* (1971) *246*:475). These results suggest that th transcriptional machinery of these genes is highly regulated, perhaps by the participation of DN sequences present in the 5' non-coding flanking region of the genes.

This invention relates to the isolation, structure and the successful use in yeast expression plasmids ⟨ DNA fragments corresponding to the 5' non-coding regions of the regulatable yeast genes GAPDH an PyK. These fragments which contain DNA sequences with strong transcription-promoting activity ar called "promoters". They are ideal components of DNA vectors for commercial production of larg quantities of protein coded by foreign genes under their transcriptional control.

In addition, this invention encompasses yeast expression plasmids further comprising an appropriat terminator to a form a "cassette" of promoter-foreign gene-terminator. The presence of the terminato increases expression of the foreign DNA.

An early attempt to express foreign DNA in yeast failed (Beggs, J. D. *et al.*, *Nature* (1980) *283*:285). I this report, the hemoglobin DNA (inserted with its own promoter) was transcribed but the RNA was no spliced. A variety of explanations for this result are possible, e.g., an incorrect location for the initiation ⟨ transcription and/or the poor ability of yeast cells to carry out splicing of intervening sequences (introns)

Three GAPDH genes of yeast have been cloned (Holland, M. J. *et al.*, *Basic Life Science* (1981) *19*:291 but their promoters have not been used for constructing expression systems in yeast by recombinant DN⟨ methods. The PyK gene has also been cloned, but by genetic complementation only (no structural studie performed) (Kawasaki, G. and Fraenel, D. G., *Biochem. Biophys. Res. Comm.* (1982) *108*:1107). Other yeas promoters, e.g., that of alcohol dehydrogenase I (Valenzuela, P. *et al.*, *Nature* (1982) *298*:347 and Hitzeman R. A. *et al.*, *Nature* (1981) *293*:717) and phosphoglycerate kinase (Tuite, M. F. *et al.*, *EMBO J.* (1982) *1*:60⟨ and Hitzeman, R. A. *et al.*, *Science* (1983) *219*:620) have been linked to foreign genes to produce yeas expression but no terminators were used. The present invention provides new promoters for yeas expression systems and combines the advantages of highly expressive promoters with the enhance⟨ expression found with appropriately ligated terminators.

A published European Patent Application (072 318) disclosed the construction of a yeast expressio⟨ vector which, upon induction, expressed hepatitis B virus surface antigen (HBsAg) S-protein under contro of the yeast alcohol dehydrogenase I (ADHI) promoter.

### Brief description of the invention

This invention relates to a yeast expression vector comprising a segment of foreign DNA, e.g., tha coding for hepatitis B virus (HBV) surface antigen (HBsAg), under transcriptional control of a yeast GAPDI promoter. Terminators may also be appropriately attached. The expression vector typically has a yeas replication origin and is capable of replicating in either type of cell. The expression vector, when used t⟨ transform yeast cells, will yield substantial amounts of the protein coded by the segment of foreign DNA

### Brief description of the drawings

Figure 1: Isolation and tailoring of a GAPDH promoter fragment.
Figure 2: DNA sequence of the GAPDH promoter fragment.
Figure 3: Construction of a yeast expression plasmid containing the GAPDH promoter.

### Detailed description of the invention

In principle, yeast expression plasmids have particular advantages, including the following. Yeast car be grown in large-scale culture for commercial production by processes well-known in the art. In contrast bacteria in large-scale culture are subject to the frequent problem of "phage-out". Yeast also appears t⟨ have much the same ability as mammalian cells to add carbohydrate groups to newly synthesized proteins a capacity that bacteria do not have. Now that cDNA sequences are readily obtainable, the problem o expressing genes having introns is easily avoided.

The vectors of the present invention encompass promoters of unusually high efficiency. A promoter is defined herein as a DNA segment capable of functioning to initiate transcription of an adjoining DNA segment. Transcription is the synthesis of RNA (herein termed messenger RNA or mRNA), complementary to one strand of the DNA adjoining the promoter region. In eukaryotes, messenger RNA synthesis is catalyzed by an enzyme termed RNA polymerase II. The minimum essential elements of promoter function are the following: To provide a starting point for the initiation of transcription and to provide a binding site for RNA polymerase II near the start site permitting selection of the proper strand of DNA as a template for messenger RNA synthesis. In addition, a eukaryotic promoter functions to regulate the relative efficiency of transcription of coding segments under its control. An active promoter is one which elicits synthesis of relatively large amounts of mRNA complementary to a strand of the adjacent DNA coding segment.

The structural correlates of promoter function have not been clearly established. A promoter segment usually can be identified in nature as a region lying adjacent to the 5' end of a given structural gene. (References to the 5' and 3' ends of a gene will be understood to indicate the corresponding respective ends of mRNA transcribed therefrom, and these, in turn, will be understood to correlate with the $NH_2$— and —COOH termini of the encoded protein, respectively). Comparisons of the nucleotide sequences of promoters for various genes from various species have revealed only a few short regions of nucleotide sequence similarity in common among them. Most notable of these is the "TATA Box," a segment of about 5 to 10 nucleotides located generally about 70 to 230 nucleotides upstream from the site of transcription initiation, having a sequence generally resembling TATAA. For review of structural comparisons see Breathnach, R. and Chambon, P., *Ann. Rev. of Biochem.* (1981) *50*:349. The TATA Box is believed to function in initiation of transcription.

The foreign gene will be free or substantially free of codons from the normal structural gene associated with the promoter. Usually, the foreign gene will be joined to a non-coding 3'-end of the regulatory region encompassing the promoter, so as to be free of the amino acids at the N-terminus of endogenous gene naturally associated with the regulatory region. That is, fewer than about 3 codons (9 nucleotides) will be retained with the regulatory region when joined to the foreign gene.

The presence of the terminator sequence at the 3' end of the coding segment enhances expression. The effect is generally similar to the addition of *rho* factor to prokaryotic transcription systems, wherein the rate of the release of RNA polymerase is enhanced to produce an increase in the rate of reinitiation of transcription. It will be understood that, while the terminator sequences are not required for detectable expression of foreign DNA segments, it is preferable to appropriately link them to enhance expression. The terminator region may be naturally associated with the same or different structural gene as the promoter region.

The most appropriate DNA vector for the GAPDH construction of this invention is a shuttle vector. These vectors can "shuttle" between a bacterial strain, such as *E. coli*, and yeast, since they have a bacterial origin of replication and a yeast origin of replication, see, e.g., Ammerer, G. *et al., Recombinant DNA, Proc. Third Cleveland Symposium Macromolecules* (Walton, A. G., ed.), p. 185, Elsevier, Amsterdam (1981). A typical bacterial origin of replication is derived from, e.g., pBR322. The most useful yeast origin of replication is found in the extrachromosomal genetic element known as the 2 micron circle. In laboratory strains the 2 micron plasmid DNA is found in approximately 50 copies per cell and is stably maintained. For a review, see, for example, *Curr. Topics Micro. Imm.* (1982) *96*:119. This yeast plasmid has also been sequenced (Hartley, J. L. *et al., Nature* (1980) *286*:860).

Representative samples of the plasmids and host cells used in the constructions of this invention have been placed on deposit with the American Type Culture Collection, 12301 Parklawn Drive, Rockville, Maryland, Plasmid pPyK 9.1.1 and yeast cell transformants 2150-2-3/pHBS-56 GAP347/33 and 2150-2-3/pHBS56PyK were placed on deposit on February 18, 1983 and have received ATCC Accession numbers 40061, 20665 and 20666, respectively.

In the Examples that follow, many of the techniques, reactions and separation procedures are already well-known in the art. All enzymes, unless otherwise stated, are available from one or more commercial sources, such as New England Biolabs, Beverly, Massachusetts; Collaborative Research, Waltham, Massachusetts; Miles Laboratories, Elkhart, Indiana; Boehringer Biochemicals, Inc., Indianapolis, Indiana and Bethesda Research Laboratories, Rockville, Maryland. Buffers and reaction conditions for restriction enzyme digestion were used according to recommendations supplied by the manufacturer for each enzyme, unless otherwise indicated. Standard methodology, for other enzyme reactions, gel electrophoresis separations and *E. coli* transformation may be found in *Methods in Enzymology*, (1979) *68*. Transformation of yeast protoplasts can be carried out essentially as described by Beggs, *Nature* (1978) *275*:104.

*E. coli* strains useful for transformation include X1776; K12 strain 294 (ATCC No. 31446); RR1 and HB101. Yeast strains XV610-8c having the genotype (*a ade2 ade6 leu2 lys1 trp1 can1*) and GM-3C-2, genotype: (*Leu2 Trp1 His4 CYC1-1CYP3-1*) (Faye, G. *et al., Proc. Natl. Acad. Sci.* (1981) *78*:/2258) can be typically used for yeast transformations. It would be understood, however, that virtually any strain of yeast is useful for transformation. Bacteria can be grown and selected according to procedures described by Miller, J. H., *Experiments in Molecular Genetics*, Cold Spring Harbor Laboratory, Cold Spring Harbor, New York (1972). Yeast can be grown on the following media: YEPD containing 1% (w/v) yeast extract, 2% (w/v) peptone and (w/v) glucose; and, in the case of plating medium, 3% (w/v) agar. YNB plus CAA contains 6.7 grams of yeast nitrogen base (Difco Laboratories, Minneapolis, Minnesota), 10 mg of adenine, 10 mg of uracil, 5 g casamino acids (CAA) (Difco), 20 g glucose; and, in the case of plating media, 30 g agar per liter.

Selection for tryptophan prototrophy can be made on plates containing 6.7 g yeast nitrogen base (lacking amino acids), supplemented for all growth requirements of the strain to be transformed except tryptophan.

Example 1
Cloning of the yeast glyceraldehyde-3-phosphate dehydrogenase (GAPDH) gene.

A complementary DNA (cDNA) containing the yeast GAPDH coding sequences was prepared in the following manner:

PolyA+ RNA was isolated from yeast strain A364A. Double-stranded cDNA was synthesized using AMV reverse transcriptase and *E. coli* DNA polymerase I. Poly-dC-tails were added to the double-stranded cDNA molecule using deoxynucleotide terminal transferase. Poly-dC-tailed cDNA was annealed to poly-dG-tailed pBR322 and used to transform *E. coli* HB101. One thousand transformants were screened by colony hybridization to labeled PolyA+ RNA, and a subset further examined by restriction endonuclease mapping, and DNA sequencing. Three clones containing GAPDH sequences were isolated from the pool. One clone (pcGAP-9) contained an insert of about 1200 base pairs (bp) and was used for further work.

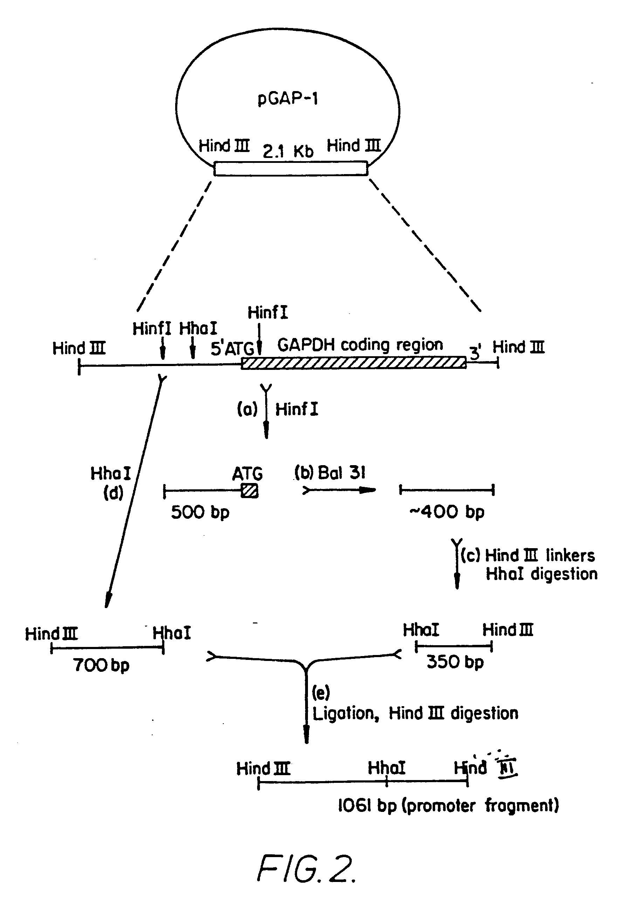

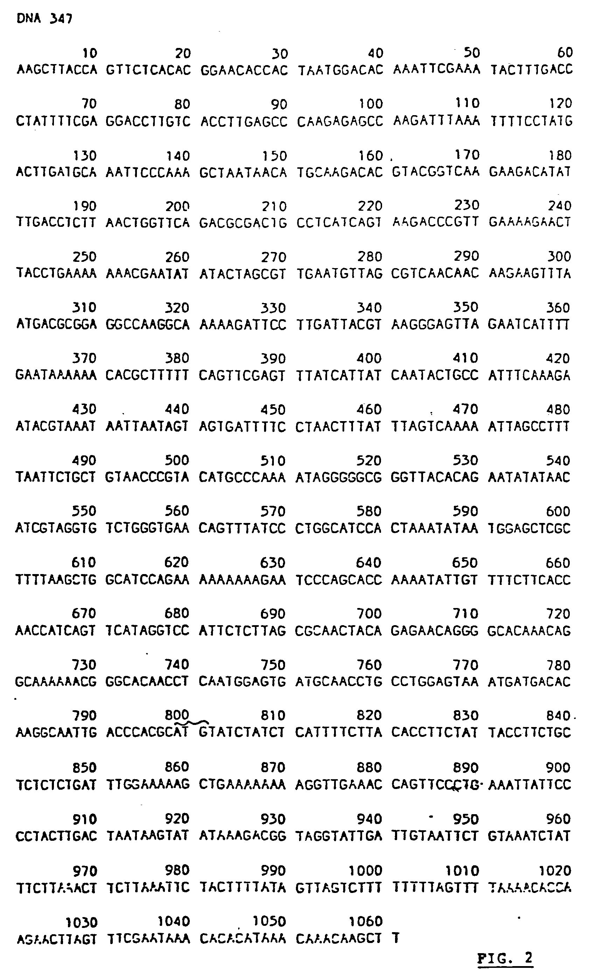

A yeast gene library was prepared by inserting fragments obtained after partial digestion of total yeast DNA with restriction endonuclease *Sau*3A into lambda phage Charon 28, according to Blattner, F. R. *et al.*, *Science* (1977) *196*:161—169. Several fragments containing yeast GAPDH coding sequences were isolated by screening the phage library with labeled DNA from pcGAP-9. The yeast GAPDH gene of one of these clones was subcloned in pBR322 as a 2.1kb *Hind*III fragment (pGAP-1, see Figure 1) or as a 3.5kb *Bam*HI fragment (pGAP-2). The GAPDH promoting-active fragments were isolated from these clones. The *Hind*III-*Hha*I fragment of about 800bp was ligated to the *Hha*I-*Hind*III fragment of about 350bp. The resulting 1061bp *Hind*III fragment was isolated by gel electrophoresis and cloned in pBR322, (pGAP-347), and the sequence determined (see Figure 2).

Example 2
Construction of yeast vectors containing the GAPDH promoter, active in the expression of HBsAg.

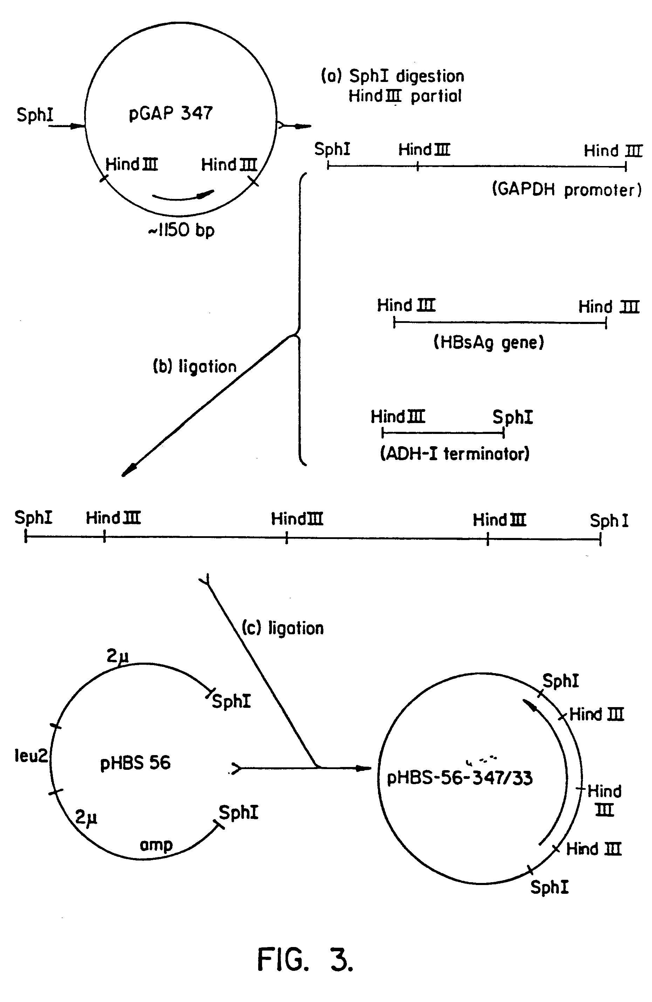

A plasmid vector (pHBS-56GAP347/33), for the expression of HBV surface antigen in yeast, using the GAPDH promoter fragment was constructed as depicted in Figure 3.

Total digestion of pGAP-347 with *Sph*I followed by partial digestion with *Hind*III yielded an approximately 1700bp *Sph*I-*Hind*III fragment having about 1060bp of GAPDH promoter and about 530bp of pBR322. The 1700bp *Sph*I-*Hind*III GAPDH promoter fragment was ligated with the 840bp *Hind*III-*Hind*III fragment (containing the HBsAg coding region, 26 bases of 5′ non-coding region and 128bp of 3′ non-coding region, obtained from pHBS-56) and then with the 350bp *Hind*III-*Sph*I fragment containing the ADH-1 termination region (isolated from pHBS-56). The 2900bp *Sph*I fragment (cassette) was isolated and cloned in pHBS-56 previously digested with *Sph*I. The plasmid pHBS-56 (ATCC Accession No. 40047) has been described in a co-pending application (EPA No. 82.401473.2 published as no. 72318, of Regents of the University of California, herein incorporated by reference) and contains the entire 2 micron plasmid, in addition to a region with the yeast leu2 gene and the amp resistance locus of pBR322. The resulting plasmid (pHBS-56GAP347/33) in which the promoter, gene and termination regions were in the proper orientations was isolated and used to transform yeast strain AB102 (*MAT*a, *pep 4-3, leu 2-3 leu2-112, ura 3-52, his 4-580, cir°*) or strain 2150-2-3 (*MAT*a, *ade1, leu2-04, cir°*). Strain AB102 is derived from SF657-9c by curing of 2 micron plasmids. Strain 2150-2-3 is from the collection of Dr. Leland Hartwell at the University of Washington.

Example 3
Synthesis of HBsAg in yeast under GAPDH promoter control (plasmid pHBS-56GAP347/33).

One hundred ml cultures of strain AB102 containing plasmid pHBS56-347/33 were grown to optical density at 650nm of 1. Cell-free lysates were prepared by agitation with glass beads and removal of cell debris by centrifugation. HBsAg was measured by the Abbott Ausriall radioimmunoassay and protein concentration was determined by the Coomassie blue binding method. The results are shown in Table 1. They indicate that the GAPDH promoter is about 5 times more effective than the ADH-1 promoter for protein product expression in yeast.

TABLE 1
Synthesis of HBsAg in yeast

(a) control from pHBS-56 (ADH-I promoter)

| Exp# | sAg (µg/ml) | Protein (mg/ml) | Spec. activity (µgsAg/mg protein) |
|---|---|---|---|
| 1 | 8.8 | 18 | 0.49 |
| 2 | 14 | 25 | 0.56 |
| 3 | 12.4 | 20 | 0.62 |

## EP 0 120 551 B1

TABLE 2 (continued)
(b) from pHBS-56GAP347/33 (GAPDH promoter)

| Exp# | sAg (µg/ml) | Protein (mg/ml) | Spec. activity (µgsAg/mg protein) |
|---|---|---|---|
| 1 | 36 | 14 | 2.6 |
| 2 | 35 | 12 | 2.9 |
| 3 | 37 | 12.5 | 3.0 |

Similar results were obtained by substituting yeast strain 2150-2-3 for yeast strain AB102 and repeating Example 3.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

### Claims for the Contracting States: BE CH DE FR GB IT LI NL LU SE

1. A yeast expression vector comprising a segment of foreign DNA under transcriptional control of a yeast glyceraldehyde-3-phosphate dehydrogenase promoter having the sequence shown in Figure 2, said segment being in the correct orientation for transcription and having fewer than three codons from yeast glyceraldehyde-3-phosphate dehydrogenase at the 5'-end of said foreign DNA.

2. A yeast expression vector of claim 1 further comprising a terminator attached to the 3' end of the segment of foreign DNA.

3. A yeast expression vector according to claim 1, further comprising yeast two micron plasmid DNA or portion thereof.

4. A yeast expression vector of claim 1 wherein said foreign DNA codes for hepatitis B surface antigen or portion thereof.

5. The plasmid pHBS-56 bAP347/33 deposited in a yeast host under the ATCC accession number 20665.

6. A method of expressing a DNA coding segment in yeast, comprising the steps of:

(a) inserting the coding segment is a yeast expression vector, said vector comprising a DNA segment derived from a yeast glyceraldehyde-3-phosphate dehydrogenase promoter having the sequence shown in Figure 2 and having fewer than three codons from the 5'-end of glyceraldehyde-3-phosphate dehydrogenase, said promoter being adjacent to the 5' end of the inserted DNA coding segment and so oriented that transcription initiated within said promoter includes the coding segment, thereby providing a coding segment expression vector, and

(b) transforming yeast cells with the coding segment expression vector.

7. A method according to claim 6 wherein said yeast expression vector further comprises a terminator attached to the 3' end of the inserted DNA coding segment.

8. A method according to claim 6 wherein said yeast expression vector further comprises a bacterial cell replication origin and is capable of replicating in a bacterial cell.

9. A method according to claim 7 wherein said terminator comprises the yeast alcohol dehydrogenase terminator.

10. A method according to claim 7 wherein said terminator comprises the yeast glyceraldehyde-3-phosphate dehydrogenase (bAPDH) terminator.

11. A method according to claim 7 wherein said terminator comprises the yeast pyruvate kinase (PyK) terminator.

### Claims for the Contracting State: AT

1. A method of expressing a DNA coding segment in yeast, comprising the steps of:

(a) inserting the coding segment in a yeast expression vector, said vector comprising a DNA segment derived from a yeast glyceraldehyde-3-phosphate dehydrogenase promoter having the sequence shown in Figure 2 and having fewer than three codons from the 5'-end of glyceraldehyde-3-phosphate dehydrogenase, said promoter being adjacent to the 5' end of the inserted DNA coding segment and so oriented that transcription initiated within said promoter includes the coding segment, thereby providing a coding segment expression vector, and

(b) transforming yeast cells with the coding segment expression vector.

5

2. A method according to claim 1 wherein said yeast expression vector further comprises a terminator attached to the 3' end of the inserted DNA coding segment.

3. A method according to claim 1 wherein said yeast expression vector further comprises a bacterial cell replication origin and is capable of replicating in a bacterial cell.

4. A method according to claim 2 wherein said terminator comprises the yeast alcohol dehydrogenase terminator.

5. A method according to claim 2 wherein said terminator comprises the yeast glyceraldehyde-3-phosphate dehydrogenase (bAPDH) terminator.

6. A method according to claim 2 wherein said terminator comprises the yeast pyruvate kinase (PyK) terminator.

7. A method according to claim 1 wherein said yeast expression vector further comprises the yeast two micron plasmid DNA or portion thereof.

8. A method according to claim 1 wherein said foreign DNA codes for hepatitis B surface antigen or portion thereof.

9. A method according to claim 1 wherein said coding expression vector is the plasmid pHBS-56 bAP347/33 deposited in a yeast host under the ATCC accession number 20665.

## Patentansprüche für die Vertragsstaaten: BE CH DE FR GB IT LI NL LU SE

1. Hefeexpressionsvektor mit einem Abschnitt einer Fremd-DNA unter Transkriptionskontrolle eines Hefe-Glyceraldehyd-3-phosphat-dehydrogenase-Promotors mit einer in Fig. 2 wiedergegebenen Sequenz, wobei sich der Abschnitt in der korrektion Orientierung für die Transkription befindet und weniger als 3 Codons von der Hefe-Glyceraldehyd-3-phosphat-dehydrogenase an dem 5'-Ende der Fremd-DNA aufweist.

2. Hefeexpressionsvektor nach Anspruch 1, der weiterhin einen am 3'-Ende des Abschnittes der Fremd-DNA angeordneten Terminator aufweist.

3. Hefeexpressionsvektor nach Anspruch 1, der weiterhin eine Hefe-2-micron-Plasmid-DNA oder einen Teil derselben aufweist.

4. Hefeexpressionsvektor nach Anspruch 1, bei dem die Fremd-DNA für ein Hepatitis B-Oberflächenantigen oder einen Teil desselben kodiert.

5. Plasmid pHBS-56GAP347/33, hinterlegt in einem Hefe-Wirtsstamm unter der ATCC-Zugangsnummer 20665.

6. Verfahren zur Expression eines DNA-Kodierungsabschnittes, umfassend die Schritte

a) Einfügung des Kodierungsabschnittes in einen Hefeexpressionsvektor, der einen von einem Hefe-Glyceraldehyd-3-phosphat-dehydrogenase-Promotor mit einer in Fig. 2 wiedergegebenen Sequenz abgeleiteten DNA-Abschnitt umfaßt und weniger als 3 Codons von dem 5'-Ende der Hefe-Glyceraldehyd-3-phosphat-hydrogenase aufweist, wobei der Promotor sich neben dem 5'-Ende des eingefügten DNA-Kodierungsabschnittes befindet und so orientiert ist, daß in dem Promotor eingeleitete Transkription den Kodierungsabschnitt einschließt und dadurch ein Kodierungsabschnitt-Expressionsvektor gebildet wird, und

b) Transformation der Hefezellen mit dem Kodierungsabschnitt-Expressionsvektor.

7. Verfahren nach Anspruch 6, bei dem der Hefeexpressionsvektor weiterhin einen am 3'-Ende des eingefügten DNA-Kodierungsabschnittes angeordneten Terminator aufweist.

8. Verfahren nach Anspruch 6, bei dem der Hefeexpressionsvektor weiterhin einen Bakterienzellen-Replikationsorigin aufweist und zur Replikation in einer Bakterienzelle befähigt ist.

9. Verfahren nach Anspruch 7, bei dem der Terminator den Hefe-Alkohol-dehydrogenase-Terminator umfaßt.

10. Verfahren nach Anspruch 7, bei dem der Terminator den Hefe-Glyceraldehyd-3-phosphat-dehydrogenase (GAPDH)-Terminator umfaßt.

11. Verfahren nach Anspruch 7, bei dem der Terminator den Hefe-Pyruvatkinase (PyK)-Terminator umfaßt.

## Patentansprüche für den Vertragsstaat: AT

1. Verfahren zur Expression eines DNA-Kodierungssegmentes in Hefe, umfassend die Schritte:

a) Einfügung des Kodierungssegmentes in einen Hefeexpressionsvektor, wobei der Vektor einen von einem Hefe-Glyceraldehyd-3-phosphat-dehydrogenase-Promotor mit einer in Fig. 2 wiedergegebenen Sequenz abgeleiteten DNA-Abschnitt umfaßt und weniger als 3 Codons von dem 5'-Ende der Hefe-Glyceraldehyd-3-phosphat-dehydrogenase aufweist, wobei der Promotor sich neben dem 5'-Ende des eingefügten DNA-Kodierungsabschnittes befindet und so orientiert ist, daß die in dem Promotor eingeleitete Transkription den Kodierungsabschnitt einschließt und dadurch ein Kodierungsabschnitt-Expressionsvektor gebildet wird, und

b) Transformation der Hefezellen mit dem Kodierungsabschnitt-Expressionsvektor.

2. Verfahren nach Anspruch 1, bei dem der Hefeexpressionsvektor weiterhin einen Terminator umfaßt, der am 3'-Ende des eingefügten DNA-Segmentes angeordnet ist.

6

3. Verfahren nach Anspruch 1, bei dem der Hefeexpressionsvektor weiterhin einen Bakterienzellen-Replikationsorigin umfaßt und in einer Bakterienzelle replizierbar ist.

4. Verfahren nach Anspruch 2, bei dem der Terminator den Hefe-Alkohol-dehydrogenase-Terminator umfaßt.

5. Verfahren nach Anspruch 2, bei dem der Terminator den Hefe-Glyceraldehyd-3-phosphat-dehydrogenase(GAPDH)-Terminator umfaßt.

6. Verfahren nach Anspruch 2, bei dem der Terminator den Hefe-Pyruvasekinase(PyK)-Terminator umfaßt.

7. Verfahren nach Anspruch 1, bei dem der Hefeexpressionsvektor weiterhin eine Hefe-2-micron-Plasmid-DNA oder Teile derselben umfaßt.

8. Verfahren nach Anspruch 1, bei dem die Fremd-DNA für Hepatitis B-Oberflächenantigen oder Teile desselben kodiert.

9. Verfahren nach Anspruch 1, bei dem der Kodierungs-Expressionsvektor das Plasmid pHBS-56GAP347/33, hinterlegt in einem Hefe-Wirtsstamm mit der Zugangsnummer ATCC 20665, ist.

## Revendications pour les Etats contractants: BE CH DE FR GB IT LI NL LU SE

1. Vecteur d'expression pour levure, comprenant un segment d'ADN étranger sous régulation de transcription d'un promoteur de glycéraldéhyde-3-phosphate-déshydrogénase de levure ayant la séquence représentée sur la fig. 2, ce segment étant dans l'orientation correcte pour la transcription et ayant moins de trois codons de glycéraldéhyde-3-phosphate-déshydrogénase de levure à l'extrémité 5' dudit ADN étranger.

2. Vecteur d'expression pour levure selon la revendication 1, comprenant en outre un terminateur attaché à l'extrémité 3' du segment d'ADN étranger.

3. Vecteur d'expression pour levure selon la revendication 1, comprenant en outre un ADN à plasmides de 2 microns de levure ou une partie de celui-ci.

4. Vecteur d'expression pour levure selon la revendication 1, dans lequel l'ADN étranger code pour l'antigène de surface de l'hépatite B ou pour une partie de celui-ci.

5. Plasmide pHBS-56 GAP 347/33 déposé dans une levure-hôte sous le numéro d'admission ATCC 20665.

6. Procédé d'expression d'un segment codant d'ADN dans une levure, comprenant les étapes consistant:

(a) à insérer le segment codant dans un vecteur d'expression de levure, ce vecteur comprenant un segment d'ADN dérivé d'un promoteur de glycéraldéhyde-3-phosphate-déshydrogénase de levure ayant la séquence représentée sur la fig. 2 et ayant moins de trois codons de l'extrémité 5' de la glycéraldéhyde-3-phosphate-déshydrogénase, ledit promoteur étant adjacent à l'extrémité 5' du segment codant d'ADN inséré et étant orienté de telle sorte que la transcription initiée dans ledit promoteur comprenne le segment codant, ce qui donne un vecteur d'expression de segment codant, et

(b) à transformer des cellules de levure avec le vecteur d'expression de segment codant.

7. Procédé selon la revendication 6, dans lequel ledit vecteur d'expression pour levure comprend en outre un terminateur attaché à l'extrémité 3' du segment codant d'ADN inséré.

8. Procédé selon la revendication 6, dans lequel ledit vecteur d'expression pour levure comprend en outre une origine de la réplication de cellules bactériennes et est capable de réplication dans une cellule bactérienne.

9. Procédé selon la revendication 7, dans lequel ledit terminateur comprend le terminateur d'alcool-déshydrogénase de levure.

10. Procédé selon la revendication 7, dans lequel ledit terminateur comprend le terminateur de glycéraldéhyde-3-phosphate-déshydrogénase (GAPDH) de levure.

11. Procédé selon la revendication 7, dans lequel ledit terminateur comprend le terminateur de pyruvatekinase (PyK) de levure.

## Revendications pour l'Etat contractant: AT

1. Procédé d'expression d'un segment codant d'ADN dans une levure, comprenant les étapes consistant:

(a) à insérer le segment codant dans un vecteur d'expression pour levure, ce vecteur comprenant un segment d'ADN dérivé d'un promoteur de glycéraldéhyde-3-phosphate-déshydrogénase de levure ayant la séquence représentée sur la fig. 2 et ayant moins de trois codons de l'extrémité 5' de la glycéraldéhyde-3-phosphate-déshydrogénase, ledit promoteur étant adjacent à l'extrémité 5' du segment codant d'ADN inséré et étant orienté de telle sorte que la transcription initiée dans ledit promoteur comprenne le segment codant, ce qui donne un vecteur d'expression de segment codant, et

(b) à transformer des cellules de levure avec le vecteur d'expression de segment codant.

2. Procédé selon la revendication 1, dans lequel ledit vecteur d'expression pour levure comprend en outre un terminateur attaché à l'extrémité 3' du segment codant d'ADN inséré.

7

3. Procédé selon la revendication 1, dans lequel ledit vecteur d'expression pour levure comprend en outre une origine de la réplication de cellules bactériennes et est capable de réplication dans une cellule bactérienne.

4. Procédé selon la revendication 2, dans lequel ledit terminateur comprend le terminateur d'alcool-déshydrogénase de levure.

5. Procédé selon la revendication 2, dans lequel ledit terminateur comprend le terminateur de glycéraldéhyde-3-phosphat-déshydrogénase (GAPDH) de levure.

6. Procédé selon la revendication 2, dans lequel ledit terminateur comprend le terminateur de pyruvatekinase (PyK) de levure.

7. Procédé selon la revendication 1, dans lequel ledit vecteur d'expression pour levure comprend en outre un ADN à plasmides de 2 microns de levure ou une partie de celui-ci.

8. Procédé selon la revendication 1, dans lequel ledit ADN étranger code pour l'antigène de surface de l'hépatite B ou pour une partie de celui-ci.

9. Procédé selon la revendication 1, dans lequel ledit vecteur d'expression codant est le plasmide pHBS-56 GAP 347/33 déposé dans une levure-hôte sous le numéro d'admission ATCC 20665.

EP 0 120 551 B1

FIG. 2.

DNA 347

```
        10         20         30         40          50         60
AAGCTTACCA GTTCTCACAC GGAACACCAC TAATGGACAC AAATTCGAAA TACTTTGACC

        70         80         90        100         110        120
CTATTTTCGA GGACCTTGTC ACCTTGAGCC CAAGAGAGCC AAGATTTAAA TTTTCCTATG

       130        140        150        160         170        180
ACTTGATGCA AATTCCCAAA GCTAATAACA TGCAAGACAC GTACGGTCAA GAAGACATAT

       190        200        210        220         230        240
TTGACCTCTT AACTGGTTCA GACGCGACTG CCTCATCAGT AAGACCCGTT GAAAAGAACT

       250        260        270        280         290        300
TACCTGAAAA AAACGAATAT ATACTAGCGT TGAATGTTAG CGTCAACAAC AAGAAGTTTA

       310        320        330        340         350        360
ATGACGCGGA GGCCAAGGCA AAAAGATTCC TTGATTACGT AAGGGAGTTA GAATCATTTT

       370        380        390        400         410        420
GAATAAAAAA CACGCTTTTT CAGTTCGAGT TTATCATTAT CAATACTGCC ATTTCAAAGA

       430        440        450        460         470        480
ATACGTAAAT AATTAATAGT AGTGATTTTC CTAACTTTAT TTAGTCAAAA ATTAGCCTTT

       490        500        510        520         530        540
TAATTCTGCT GTAACCCGTA CATGCCCAAA ATAGGGGGCG GGTTACACAG AATATATAAC

       550        560        570        580         590        600
ATCGTAGGTG TCTGGGTGAA CAGTTTATCC CTGGCATCCA CTAAATATAA TGGAGCTCGC

       610        620        630        640         650        660
TTTTAAGCTG GCATCCAGAA AAAAAAGAA TCCCAGCACC AAAATATTGT TTTCTTCACC

       670        680        690        700         710        720
AACCATCAGT TCATAGGTCC ATTCTCTTAG CGCAACTACA GAGAACAGGG GCACAAACAG

       730        740        750        760         770        780
GCAAAAACG GGCACAACCT CAATGGAGTG ATGCAACCTG CCTGGAGTAA ATGATGACAC

       790        800        810        820         830        840
AAGGCAATTG ACCCACGCAT GTATCTATCT CATTTTCTTA CACCTTCTAT TACCTTCTGC

       850        860        870        880         890        900
TCTCTCTGAT TTGGAAAAAG CTGAAAAAAA AGGTTGAAAC CAGTTCCCTG AAATTATTCC

       910        920        930        940          950        960
CCTACTTGAC TAATAAGTAT ATAAAGACGG TAGGTATTGA TTGTAATTCT GTAAATCTAT

       970        980        990       1000        1010       1020
TTCTTAAACT TCTTAAATTC TACTTTTATA GTTAGTCTTT TTTTTAGTTT TAAAACACCA

      1030       1040       1050       1060
AGAACTTAGT TTCGAATAAA CACACATAAA CAAACAAGCT T
```

FIG. 2

2

FIG. 3.